# EUROPEAN PATENT SPECIFICATION

(11) **EP 3 156 798 B1**
(45) Date of publication and mention of the grant of the patent: **12.12.2018**
(21) Application number: 15002958.5
(22) Date of filing: 16.10.2015
(51) Int. Cl.: G01N 33/53

(54) **A NOVEL ASSAY FOR THE DIAGNOSIS OF HELMINTH INFECTIONS**
NEUARTIGER ASSAY ZUR DIAGNOSE VON HELMINTHINFEKTIONEN
NOUVEAU DOSAGE POUR LE DIAGNOSTIC D'INFECTIONS HELMINTHIQUES

(43) Date of publication of application: 19.04.2017
(73) Proprietor: Euroimmun Medizinische Labordiagnostika AG, 23560 Lübeck (DE); Universität Bern, 3012 Bern (CH)
(72) Inventor: Scheper, Thomas, 23560 Lübeck (DE); Meyer, Wolfgang, 23560 Lübeck (DE); Schönfeld, Linda, 23560 Lübeck (DE); Gottstein, Bruno, 3012 Bern (CH); Spiliotis, Markus, 3012 Bern (CH)

(56) References cited:
- FR-A1- 2 863 499
- WEN H1 ET AL: "Immunoblot evaluation of IgG and IgG-subclass antibody responses for immunodiagnosis of human alveolar echinococcosis", ANNALS OF TROPICAL MEDICINE AND PARASITOLOGY, ACADEMIC PRESS, LONDON, GB, vol. 89, no. 5, 1 October 1995 (1995-10-01), pages 485-495, XP009188622, ISSN: 0003-4983
- A Ito ET AL: "Differential serodiagnosis for cystic and alveolar echinococcosis using fractions of Echinococcus granulosus cyst fluid (antigen B) and E. multilocularis protoscolex (EM18)", American Journal of Tropical Medicine and Hygiene, 1 February 1999 (1999-02-01), pages 188-192, XP055251132, UNITED STATES Retrieved from the Internet: URL:http://www.ajtmh.org/content/60/2/188. full.pdf [retrieved on 2016-02-18]
- SCHWEIGER A ET AL: "Serological diagnosis of echinococcosis: the diagnostic potential of native antigens", INFECTION ; A JOURNAL OF INFECTIOUS DISEASE, URBAN & VOGEL, MU, vol. 40, no. 2, 11 November 2011 (2011-11-11), pages 139-152, XP035050776, ISSN: 1439-0973, DOI: 10.1007/S15010-011-0205-6
- AHN CHUN-SEOB ET AL: "An Echinococcus multilocularis Antigen B3 Proteoform That Shows Specific Antibody Responses to Active-Stage Alveolar Echinococcosis.", JOURNAL OF CLINICAL MICROBIOLOGY OCT 2015, vol. 53, no. 10, October 2015 (2015-10), pages 3310-3317, XP9188600, ISSN: 1098-660X

### SEQUENCE LISTING

<110> EUROIMMUN
<120> A novel assay for the diagnosis of helminth infections
<130> p00056
<160> 17
<170> BiSSAP 1.3
<210> 1
   <211> 66
   <212> PRT
   <213> Echinococcus granulosus
<220>
   <223> Eg AgB8/1a
<400> 1
   Met Asp Asp Gly Leu Thr Ser Thr Ser Arg Ser Val Met Lys Met Phe
   1               5                   10                  15
   Gly Glu Val Lys Tyr Phe Phe Glu Arg Asp Pro Leu Gly Gln Lys Val
               20                  25                  30
   Val Asp Leu Leu Lys Glu Leu Glu Glu Val Phe Gln Leu Leu Arg Lys
           35                  40                  45
   Lys Leu Arg Met Ala Leu Arg Ser His Leu Arg Gly Leu Ile Ala Glu
       50                  55                  60
   Gly Glu
   65
<210> 2
   <211> 66
   <212> PRT
   <213> Echinococcus granulosus
<220>
   <223> EG AgB8/1b
<400> 2
   Met Asp Asp Gly Leu Thr Ser Thr Ser Arg Ser Val Met Lys Met Ile
   1               5                   10                  15
   Gly Glu Arg Lys Tyr Phe Phe Glu Arg Asp Pro Leu Gly Gln Lys Val
               20                  25                  30
   Val Asp Leu Leu Lys Glu Leu Glu Glu Val Phe Gln Leu Leu Arg Lys
           35                  40                  45
   Lys Leu Arg Thr Ala Leu Lys Ser His Leu Arg Glu Leu Val Ala Glu
       50                  55                  60
   Gly Lys
   65
<210> 3
   <211> 71
   <212> PRT
   <213> Echinococcus granulosus
<220>
   <223> Eg AgB8/2
<400> 3
   Met Lys Asp Glu Pro Lys Ala His Met Gly Gln Val Val Lys Lys Arg
   1               5                   10                  15
   Trp Gly Glu Leu Arg Asp Phe Phe Arg Asn Asp Pro Leu Gly Gln Arg
               20                  25                  30
   Leu Val Ala Leu Gly Asn Asp Leu Thr Ala Ile Cys Gln Lys Leu Gln
           35                  40                  45
   Leu Lys Ile Arg Glu Val Leu Lys Lys Tyr Val Lys Asn Leu Val Glu
       50                  55                  60
   Glu Lys Asp Asp Asp Ser Lys
   65                  70
<210> 4
   <211> 70
   <212> PRT
   <213> Echinococcus granulosus
<220>
   <223> Eg AgB8/3
<400> 4
   Met Asp Asp Asp Asp Asp Asp Glu Val Thr Lys Thr Lys Lys Gly Val
   1 5 10 15
   Met Lys Ala Ile Ser Glu Ile Lys His Phe Phe Gln Ser Asp Pro Leu
   20 25 30
   Gly Lys Lys Leu Val Glu Val Met Lys Asp Val Ala Ser Val Cys Glu
   35 40 45
   Met Val Arg Lys Lys Ala Arg Met Ala Leu Lys Glu Tyr Val Arg Lys
   50 55 60
   Leu Val Lys Glu Asp Glu
   65 70
<210> 5
   <211> 71
   <212> PRT
   <213> Echinococcus granulosus
<220>
   <223> Eg AgB8/4a
<400> 5
   Met Lys Ala Glu Pro Glu Arg Cys Lys Cys Leu Ile Met Arg Lys Leu
   1 5 10 15
   Gly Glu Ile Arg Asp Phe Phe Arg Ser Asp Pro Leu Gly Gln Lys Leu
   20 25 30
   Ala Ala Leu Gly Arg Asp Leu Thr Ala Ile Cys Gln Lys Leu Gln Leu
   35 40 45
   Lys Val His Glu Val Leu Lys Lys Tyr Val Lys Asp Leu Leu Glu Glu
   50 55 60
   Glu Asp Glu Asp Asp Leu Lys
   65 70
<210> 6
   <211> 72
   <212> PRT
   <213> Echinococcus granulosus
<220>
   <223> Eg_AgB8/4b
<400> 6
   Met Lys Ala Glu Pro Glu Arg Cys Lys Cys Leu Ile Thr Arg Lys Leu
   1 5 10 15
   Ser Glu Val Arg Asp Phe Phe Arg Ser Asp Pro Leu Gly Gln Arg Leu
   20 25 30
   Val Ala Leu Gly Arg Asp Leu Thr Ala Ile Cys Gln Lys Leu His Leu
   35 40 45
   Lys Ile His Glu Val Leu Lys Lys Tyr Val Lys Asp Leu Leu Glu Glu
   50 55 60
   Glu Glu Glu Glu Asp Asp Ser Lys
   65 70
<210> 7
   <211> 186
   <212> PRT
   <213> Echinococcus multilocularis
<220>
   <223> Em18
<400> 7
   Glu Gln Lys Leu Arg Glu Leu Arg Ala Gln Met Val Glu Lys Glu Ser
   1               5                   10                  15
   Asp Leu Ala Asp Met Lys Asn Lys Ala Ser Ala Tyr Glu Ser Lys Ile
               20                  25                  30
   Ala Glu Leu Glu Met Leu Leu Gln Gln Glu Arg His Ala Arg Glu Ser
           35                  40                  45
   Leu Gln Lys Ser Gln Asp Lys Leu Ala Glu Met Asn Arg Lys Leu Lys
       50                  55                  60
   Glu Glu Thr Ala Ala Ser Ala Glu Glu Arg Asp Arg Leu Met Ala Gln
   65                  70                  75                  80
   Arg Asp Glu Val Gln Arg Glu Val Glu Ala Gln Lys Val Ala Met Ala
                   85                  90                  95
   Lys Lys Glu Ala Glu Lys Ala Gln Ala Glu Ala Glu Leu Arg Arg Met
               100                 105                 110
   Arg Glu Lys His Asp Ala Lys His Lys Ser Gln Val Asn Gly Ser Gly
           115                 120                 125
   Asp Ala Ala Ser Gln Asp Asp Glu Ser Glu Ala Lys Glu Leu Glu Val
       130                 135                 140
   Ile Pro Asn Val Arg Arg Thr Glu Glu Ser Arg Val Thr Ala Val Ser
   145                 150                 155                 160
   Lys Asn Glu Thr Leu Gln Thr Lys Leu Ala Asn Leu Lys Met Glu Leu
                   165                 170                 175
   Ser Ser Thr Arg Asp Gln Ser Lys Met Arg
               180                 185
<210> 8
   <211> 140
   <212> PRT
   <213> Echinococcus multilocularis
<220>
   <223> Em95
<400> 8
   Gln Glu Tyr Arg Gly Arg Gly Ile Glu Ile Lys Thr Thr Glu Ser Pro
   1               5                   10                  15
   Leu Arg Lys His Phe Ser Leu Thr Leu Val Gly Ser Gln Gly Ile Arg
               20                  25                  30
   Leu Ser Trp Asp Val Gln His Leu Pro Asp Leu Arg Gly Thr Asn Ile
           35                  40                  45
   Ser Leu Lys Ala Leu Asp Pro Ser Asp Pro Leu Val Tyr Lys Arg Gln
       50                  55                  60
   Thr Ala Gln Phe Ser Asp Gly Gln Leu Ala Ile Gly Arg Leu Lys Pro
   65                  70                  75                  80
   Ser Thr Leu Tyr Gln Met Thr Val Glu Ala Val Arg Gly Lys Asn Thr
                   85                  90                  95
   Thr Leu Lys Phe Thr Glu Asp Ile Lys Thr Leu Arg Ile Gly Glu Lys
               100                 105                 110
   Glu Ser Thr Val Met Thr Ser Gly Ser Ala Leu Thr Ser Ala Ile Ala
           115                 120                 125
   Gly Phe Val Phe Ser Cys Ile Val Ile Val Leu Thr
       130                 135                 140
<210> 9
   <211> 423
   <212> PRT
   <213> Artificial Sequence
<220>
   <223> AgB8x
<400> 9
   Ser His His His His His His His His Ser Met Asp Asp Gly Leu Thr
   1               5                   10                  15
   Ser Thr Ser Arg Ser Val Met Lys Met Phe Gly Glu Val Lys Tyr Phe
               20                  25                  30
   Phe Glu Arg Asp Pro Leu Gly Gln Lys Val Val Asp Leu Leu Lys Glu
           35                  40                  45
   Leu Glu Glu Val Phe Gln Leu Leu Arg Lys Lys Leu Arg Met Ala Leu
       50                  55                  60
   Arg Ser His Leu Arg Gly Leu Ile Ala Glu Gly Glu Asp Asp Gly Leu
   65                  70                  75                  80
   Thr Ser Thr Ser Arg Ser Val Met Lys Met Ile Gly Glu Arg Lys Tyr
                   85                  90                  95
   Phe Phe Glu Arg Asp Pro Leu Gly Gln Lys Val Val Asp Leu Leu Lys
               100                 105                 110
   Glu Leu Glu Glu Val Phe Gln Leu Leu Arg Lys Lys Leu Arg Thr Ala
           115                 120                 125
   Leu Lys Ser His Leu Arg Glu Leu Val Ala Glu Gly Lys Lys Asp Glu
       130                 135                 140
   Pro Lys Ala His Met Gly Gln Val Val Lys Lys Arg Trp Gly Glu Leu
   145                 150                 155                 160
   Arg Asp Phe Phe Arg Asn Asp Pro Leu Gly Gln Arg Leu Val Ala Leu
                   165                 170                 175
   Gly Asn Asp Leu Thr Ala Ile Cys Gln Lys Leu Gln Leu Lys Ile Arg
               180                 185                 190
   Glu Val Leu Lys Lys Tyr Val Lys Asn Leu Val Glu Glu Lys Asp Asp
           195                 200                 205
   Asp Ser Lys Asp Asp Asp Asp Asp Asp Glu Val Thr Lys Thr Lys Lys
       210                 215                 220
   Gly Val Met Lys Ala Ile Ser Glu Ile Lys His Phe Phe Gln Ser Asp
   225                 230                 235                 240
   Pro Leu Gly Lys Lys Leu Val Glu Val Met Lys Asp Val Ala Ser Val
                   245                 250                 255
   Cys Glu Met Val Arg Lys Lys Ala Arg Met Ala Leu Lys Glu Tyr Val
               260                 265                 270
   Arg Lys Leu Val Lys Glu Asp Glu Lys Ala Glu Pro Glu Arg Cys Lys
           275                 280                 285
   Cys Leu Ile Met Arg Lys Leu Gly Glu Ile Arg Asp Phe Phe Arg Ser
       290                 295                 300
   Asp Pro Leu Gly Gln Lys Leu Ala Ala Leu Gly Arg Asp Leu Thr Ala
   305                 310                 315                 320
   Ile Cys Gln Lys Leu Gln Leu Lys Val His Glu Val Leu Lys Lys Tyr
                   325                 330                 335
   Val Lys Asp Leu Leu Glu Glu Glu Asp Glu Asp Asp Leu Lys Lys Ala
               340                 345                 350
   Glu Pro Glu Arg Cys Lys Cys Leu Ile Thr Arg Lys Leu Ser Glu Val
           355                 360                 365
   Arg Asp Phe Phe Arg Ser Asp Pro Leu Gly Gln Arg Leu Val Ala Leu
       370                 375                 380
   Gly Arg Asp Leu Thr Ala Ile Cys Gln Lys Leu His Leu Lys Ile His
   385                 390                 395                 400
   Glu Val Leu Lys Lys Tyr Val Lys Asp Leu Leu Glu Glu Glu Glu Glu
                   405                 410                 415
   Glu Asp Asp Ser Lys Leu Glu
               420
<210> 10
   <211> 302
   <212> PRT
   <213> Artificial Sequence
<220>
   <223> His-GST-(PSc)-Eg_AgB8/1a
<400> 10
   Ser His His His His His His His His Met Ser Pro Ile Leu Gly Tyr
   1 5 10 15
   Trp Lys Ile Lys Gly Leu Val Gln Pro Thr Arg Leu Leu Leu Glu Tyr
   20 25 30
   Leu Glu Glu Lys Tyr Glu Glu His Leu Tyr Glu Arg Asp Glu Gly Asp
   35 40 45
   Lys Trp Arg Asn Lys Lys Phe Glu Leu Gly Leu Glu Phe Pro Asn Leu
   50 55 60
   Pro Tyr Tyr Ile Asp Gly Asp Val Lys Leu Thr Gln Ser Met Ala Ile
   65 70 75 80
   Ile Arg Tyr Ile Ala Asp Lys His Asn Met Leu Gly Gly Cys Pro Lys
   85 90 95
   Glu Arg Ala Glu Ile Ser Met Leu Glu Gly Ala Val Leu Asp Ile Arg
   100 105 110
   Tyr Gly Val Ser Arg Ile Ala Tyr Ser Lys Asp Phe Glu Thr Leu Lys
   115 120 125
   Val Asp Phe Leu Ser Lys Leu Pro Glu Met Leu Lys Met Phe Glu Asp
   130 135 140
   Arg Leu Cys His Lys Thr Tyr Leu Asn Gly Asp His Val Thr His Pro
   145 150 155 160
   Asp Phe Met Leu Tyr Asp Ala Leu Asp Val Val Leu Tyr Met Asp Pro
   165 170 175
   Met Cys Leu Asp Ala Phe Pro Lys Leu Val Cys Phe Lys Lys Arg Ile
   180 185 190
   Glu Ala Ile Pro Gln Ile Asp Lys Tyr Leu Lys Ser Ser Lys Tyr Ile
   195 200 205
   Ala Trp Pro Leu Gln Gly Trp Gln Ala Thr Phe Gly Gly Gly Asp His
   210 215 220
   Pro Pro Lys Leu Glu Val Leu Phe Gln Gly Pro Ala Met Asp Asp Gly
   225 230 235 240
   Leu Thr Ser Thr Ser Arg Ser Val Met Lys Met Phe Gly Glu Val Lys
   245 250 255
   Tyr Phe Phe Glu Arg Asp Pro Leu Gly Gln Lys Val Val Asp Leu Leu
   260 265 270
   Lys Glu Leu Glu Glu Val Phe Gln Leu Leu Arg Lys Lys Leu Arg Met
   275 280 285
   Ala Leu Arg Ser His Leu Arg Gly Leu Ile Ala Glu Gly Glu
   290 295 300
<210> 11
   <211> 302
   <212> PRT
   <213> Artificial Sequence
<220>
   <223> His-GST-(PSc)-Eg_AgB8/1b
<400> 11
   Ser His His His His His His His His Met Ser Pro Ile Leu Gly Tyr
   1               5                   10                  15
   Trp Lys Ile Lys Gly Leu Val Gln Pro Thr Arg Leu Leu Leu Glu Tyr
               20                  25                  30
   Leu Glu Glu Lys Tyr Glu Glu His Leu Tyr Glu Arg Asp Glu Gly Asp
           35                  40                  45
   Lys Trp Arg Asn Lys Lys Phe Glu Leu Gly Leu Glu Phe Pro Asn Leu
       50                  55                  60
   Pro Tyr Tyr Ile Asp Gly Asp Val Lys Leu Thr Gln Ser Met Ala Ile
   65                  70                  75                  80
   Ile Arg Tyr Ile Ala Asp Lys His Asn Met Leu Gly Gly Cys Pro Lys
                   85                  90                  95
   Glu Arg Ala Glu Ile Ser Met Leu Glu Gly Ala Val Leu Asp Ile Arg
               100                 105                 110
   Tyr Gly Val Ser Arg Ile Ala Tyr Ser Lys Asp Phe Glu Thr Leu Lys
           115                 120                 125
   Val Asp Phe Leu Ser Lys Leu Pro Glu Met Leu Lys Met Phe Glu Asp
       130                 135                 140
   Arg Leu Cys His Lys Thr Tyr Leu Asn Gly Asp His Val Thr His Pro
   145                 150                 155                 160
   Asp Phe Met Leu Tyr Asp Ala Leu Asp Val Val Leu Tyr Met Asp Pro
                   165                 170                 175
   Met Cys Leu Asp Ala Phe Pro Lys Leu Val Cys Phe Lys Lys Arg Ile
               180                 185                 190
   Glu Ala Ile Pro Gln Ile Asp Lys Tyr Leu Lys Ser Ser Lys Tyr Ile
           195                 200                 205
   Ala Trp Pro Leu Gln Gly Trp Gln Ala Thr Phe Gly Gly Gly Asp His
       210                 215                 220
   Pro Pro Lys Leu Glu Val Leu Phe Gln Gly Pro Ala Met Asp Asp Gly
   225                 230                 235                 240
   Leu Thr Ser Thr Ser Arg Ser Val Met Lys Met Ile Gly Glu Arg Lys
                   245                 250                 255
   Tyr Phe Phe Glu Arg Asp Pro Leu Gly Gln Lys Val Val Asp Leu Leu
               260                 265                 270
   Lys Glu Leu Glu Glu Val Phe Gln Leu Leu Arg Lys Lys Leu Arg Thr
           275                 280                 285
   Ala Leu Lys Ser His Leu Arg Glu Leu Val Ala Glu Gly Lys
       290                 295                 300
<210> 12
   <211> 307
   <212> PRT
   <213> Artificial Sequence
<220>
   <223> His-GST-(PSc)-Eg_AgB8/2
<400> 12
   Ser His His His His His His His His Met Ser Pro Ile Leu Gly Tyr
   1 5 10 15
   Trp Lys Ile Lys Gly Leu Val Gln Pro Thr Arg Leu Leu Leu Glu Tyr
   20 25 30
   Leu Glu Glu Lys Tyr Glu Glu His Leu Tyr Glu Arg Asp Glu Gly Asp
   35 40 45
   Lys Trp Arg Asn Lys Lys Phe Glu Leu Gly Leu Glu Phe Pro Asn Leu
   50 55 60
   Pro Tyr Tyr Ile Asp Gly Asp Val Lys Leu Thr Gln Ser Met Ala Ile
   65 70 75 80
   Ile Arg Tyr Ile Ala Asp Lys His Asn Met Leu Gly Gly Cys Pro Lys
   85 90 95
   Glu Arg Ala Glu Ile Ser Met Leu Glu Gly Ala Val Leu Asp Ile Arg
   100 105 110
   Tyr Gly Val Ser Arg Ile Ala Tyr Ser Lys Asp Phe Glu Thr Leu Lys
   115 120 125
   Val Asp Phe Leu Ser Lys Leu Pro Glu Met Leu Lys Met Phe Glu Asp
   130 135 140
   Arg Leu Cys His Lys Thr Tyr Leu Asn Gly Asp His Val Thr His Pro
   145 150 155 160
   Asp Phe Met Leu Tyr Asp Ala Leu Asp Val Val Leu Tyr Met Asp Pro
   165 170 175
   Met Cys Leu Asp Ala Phe Pro Lys Leu Val Cys Phe Lys Lys Arg Ile
   180 185 190
   Glu Ala Ile Pro Gln Ile Asp Lys Tyr Leu Lys Ser Ser Lys Tyr Ile
   195 200 205
   Ala Trp Pro Leu Gln Gly Trp Gln Ala Thr Phe Gly Gly Gly Asp His
   210 215 220
   Pro Pro Lys Leu Glu Val Leu Phe Gln Gly Pro Ala Met Lys Asp Glu
   225 230 235 240
   Pro Lys Ala His Met Gly Gln Val Val Lys Lys Arg Trp Gly Glu Leu
   245 250 255
   Arg Asp Phe Phe Arg Asn Asp Pro Leu Gly Gln Arg Leu Val Ala Leu
   260 265 270
   Gly Asn Asp Leu Thr Ala Ile Cys Gln Lys Leu Gln Leu Lys Ile Arg
   275 280 285
   Glu Val Leu Lys Lys Tyr Val Lys Asn Leu Val Glu Glu Lys Asp Asp
   290 295 300
   Asp Ser Lys
   305
<210> 13
   <211> 306
   <212> PRT
   <213> Artificial Sequence
<220>
   <223> His-GST-(PSc)-Eg_AgB8/3
<400> 13
   Ser His His His His His His His His Met Ser Pro Ile Leu Gly Tyr
   1               5                   10                  15
   Trp Lys Ile Lys Gly Leu Val Gln Pro Thr Arg Leu Leu Leu Glu Tyr
               20                  25                  30
   Leu Glu Glu Lys Tyr Glu Glu His Leu Tyr Glu Arg Asp Glu Gly Asp
           35                  40                  45
   Lys Trp Arg Asn Lys Lys Phe Glu Leu Gly Leu Glu Phe Pro Asn Leu
       50                  55                  60
   Pro Tyr Tyr Ile Asp Gly Asp Val Lys Leu Thr Gln Ser Met Ala Ile
   65                  70                  75                  80
   Ile Arg Tyr Ile Ala Asp Lys His Asn Met Leu Gly Gly Cys Pro Lys
                   85                  90                  95
   Glu Arg Ala Glu Ile Ser Met Leu Glu Gly Ala Val Leu Asp Ile Arg
               100                 105                 110
   Tyr Gly Val Ser Arg Ile Ala Tyr Ser Lys Asp Phe Glu Thr Leu Lys
           115                 120                 125
   Val Asp Phe Leu Ser Lys Leu Pro Glu Met Leu Lys Met Phe Glu Asp
       130                 135                 140
   Arg Leu Cys His Lys Thr Tyr Leu Asn Gly Asp His Val Thr His Pro
   145                 150                 155                 160
   Asp Phe Met Leu Tyr Asp Ala Leu Asp Val Val Leu Tyr Met Asp Pro
                   165                 170                 175
   Met Cys Leu Asp Ala Phe Pro Lys Leu Val Cys Phe Lys Lys Arg Ile
               180                 185                 190
   Glu Ala Ile Pro Gln Ile Asp Lys Tyr Leu Lys Ser Ser Lys Tyr Ile
           195                 200                 205
   Ala Trp Pro Leu Gln Gly Trp Gln Ala Thr Phe Gly Gly Gly Asp His
       210                 215                 220
   Pro Pro Lys Leu Glu Val Leu Phe Gln Gly Pro Ala Met Asp Asp Asp
   225                 230                 235                 240
   Asp Asp Asp Glu Val Thr Lys Thr Lys Lys Gly Val Met Lys Ala Ile
                   245                 250                 255
   Ser Glu Ile Lys His Phe Phe Gln Ser Asp Pro Leu Gly Lys Lys Leu
               260                 265                 270
   Val Glu Val Met Lys Asp Val Ala Ser Val Cys Glu Met Val Arg Lys
           275                 280                 285
   Lys Ala Arg Met Ala Leu Lys Glu Tyr Val Arg Lys Leu Val Lys Glu
       290                 295                 300
   Asp Glu
   305
<210> 14
   <211> 307
   <212> PRT
   <213> Artificial Sequence
<220>
   <223> His-GST-(PSc)-Eg_AgB8/4a
<400> 14
   Ser His His His His His His His His Met Ser Pro Ile Leu Gly Tyr
   1               5                   10                  15
   Trp Lys Ile Lys Gly Leu Val Gln Pro Thr Arg Leu Leu Leu Glu Tyr
               20                  25                  30
   Leu Glu Glu Lys Tyr Glu Glu His Leu Tyr Glu Arg Asp Glu Gly Asp
           35                  40                  45
   Lys Trp Arg Asn Lys Lys Phe Glu Leu Gly Leu Glu Phe Pro Asn Leu
       50                  55                  60
   Pro Tyr Tyr Ile Asp Gly Asp Val Lys Leu Thr Gln Ser Met Ala Ile
   65                  70                  75                  80
   Ile Arg Tyr Ile Ala Asp Lys His Asn Met Leu Gly Gly Cys Pro Lys
                   85                  90                  95
   Glu Arg Ala Glu Ile Ser Met Leu Glu Gly Ala Val Leu Asp Ile Arg
               100                 105                 110
   Tyr Gly Val Ser Arg Ile Ala Tyr Ser Lys Asp Phe Glu Thr Leu Lys
           115                 120                 125
   Val Asp Phe Leu Ser Lys Leu Pro Glu Met Leu Lys Met Phe Glu Asp
       130                 135                 140
   Arg Leu Cys His Lys Thr Tyr Leu Asn Gly Asp His Val Thr His Pro
   145                 150                 155                 160
   Asp Phe Met Leu Tyr Asp Ala Leu Asp Val Val Leu Tyr Met Asp Pro
                   165                 170                 175
   Met Cys Leu Asp Ala Phe Pro Lys Leu Val Cys Phe Lys Lys Arg Ile
               180                 185                 190
   Glu Ala Ile Pro Gln Ile Asp Lys Tyr Leu Lys Ser Ser Lys Tyr Ile
           195                 200                 205
   Ala Trp Pro Leu Gln Gly Trp Gln Ala Thr Phe Gly Gly Gly Asp His
       210                 215                 220
   Pro Pro Lys Leu Glu Val Leu Phe Gln Gly Pro Ala Met Lys Ala Glu
   225                 230                 235                 240
   Pro Glu Arg Cys Lys Cys Leu Ile Met Arg Lys Leu Gly Glu Ile Arg
                   245                 250                 255
   Asp Phe Phe Arg Ser Asp Pro Leu Gly Gln Lys Leu Ala Ala Leu Gly
               260                 265                 270
   Arg Asp Leu Thr Ala Ile Cys Gln Lys Leu Gln Leu Lys Val His Glu
           275                 280                 285
   Val Leu Lys Lys Tyr Val Lys Asp Leu Leu Glu Glu Glu Asp Glu Asp
       290                 295                 300
   Asp Leu Lys
   305
<210> 15
   <211> 308
   <212> PRT
   <213> Artificial Sequence
<220>
   <223> His-GST-(PSc)-Eg_AgB8/4b
<400> 15
   Ser His His His His His His His His Met Ser Pro Ile Leu Gly Tyr
   1               5                   10                  15
   Trp Lys Ile Lys Gly Leu Val Gln Pro Thr Arg Leu Leu Leu Glu Tyr
               20                  25                  30
   Leu Glu Glu Lys Tyr Glu Glu His Leu Tyr Glu Arg Asp Glu Gly Asp
           35                  40                  45
   Lys Trp Arg Asn Lys Lys Phe Glu Leu Gly Leu Glu Phe Pro Asn Leu
       50                  55                  60
   Pro Tyr Tyr Ile Asp Gly Asp Val Lys Leu Thr Gln Ser Met Ala Ile
   65                  70                  75                  80
   Ile Arg Tyr Ile Ala Asp Lys His Asn Met Leu Gly Gly Cys Pro Lys
                   85                  90                  95
   Glu Arg Ala Glu Ile Ser Met Leu Glu Gly Ala Val Leu Asp Ile Arg
               100                 105                 110
   Tyr Gly Val Ser Arg Ile Ala Tyr Ser Lys Asp Phe Glu Thr Leu Lys
           115                 120                 125
   Val Asp Phe Leu Ser Lys Leu Pro Glu Met Leu Lys Met Phe Glu Asp
       130                 135                 140
   Arg Leu Cys His Lys Thr Tyr Leu Asn Gly Asp His Val Thr His Pro
   145                 150                 155                 160
   Asp Phe Met Leu Tyr Asp Ala Leu Asp Val Val Leu Tyr Met Asp Pro
                   165                 170                 175
   Met Cys Leu Asp Ala Phe Pro Lys Leu Val Cys Phe Lys Lys Arg Ile
               180                 185                 190
   Glu Ala Ile Pro Gln Ile Asp Lys Tyr Leu Lys Ser Ser Lys Tyr Ile
           195                 200                 205
   Ala Trp Pro Leu Gln Gly Trp Gln Ala Thr Phe Gly Gly Gly Asp His
       210                 215                 220
   Pro Pro Lys Leu Glu Val Leu Phe Gln Gly Pro Ala Met Lys Ala Glu
   225                 230                 235                 240
   Pro Glu Arg Cys Lys Cys Leu Ile Thr Arg Lys Leu Ser Glu Val Arg
                   245                 250                 255
   Asp Phe Phe Arg Ser Asp Pro Leu Gly Gln Arg Leu Val Ala Leu Gly
               260                 265                 270
   Arg Asp Leu Thr Ala Ile Cys Gln Lys Leu His Leu Lys Ile His Glu
           275                 280                 285
   Val Leu Lys Lys Tyr Val Lys Asp Leu Leu Glu Glu Glu Glu Glu Glu
   290 295 300
   Asp Asp Ser Lys
   305
<210> 16
   <211> 194
   <212> PRT
   <213> Artificial Sequence
<220>
   <223> Em18-His
<400> 16
   Glu Gln Lys Leu Arg Glu Leu Arg Ala Gln Met Val Glu Lys Glu Ser
   1 5 10 15
   Asp Leu Ala Asp Met Lys Asn Lys Ala Ser Ala Tyr Glu Ser Lys Ile
   20 25 30
   Ala Glu Leu Glu Met Leu Leu Gln Gln Glu Arg His Ala Arg Glu Ser
   35 40 45
   Leu Gln Lys Ser Gln Asp Lys Leu Ala Glu Met Asn Arg Lys Leu Lys
   50 55 60
   Glu Glu Thr Ala Ala Ser Ala Glu Glu Arg Asp Arg Leu Met Ala Gln
   65 70 75 80
   Arg Asp Glu Val Gln Arg Glu Val Glu Ala Gln Lys Val Ala Met Ala
   85 90 95
   Lys Lys Glu Ala Glu Lys Ala Gln Ala Glu Ala Glu Leu Arg Arg Met
   100 105 110
   Arg Glu Lys His Asp Ala Lys His Lys Ser Gln Val Asn Gly Ser Gly
   115 120 125
   Asp Ala Ala Ser Gln Asp Asp Glu Ser Glu Ala Lys Glu Leu Glu Val
   130 135 140
   Ile Pro Asn Val Arg Arg Thr Glu Glu Ser Arg Val Thr Ala Val Ser
   145 150 155 160
   Lys Asn Glu Thr Leu Gln Thr Lys Leu Ala Asn Leu Lys Met Glu Leu
   165 170 175
   Ser Ser Thr Arg Asp Gln Ser Lys Met Arg Leu Glu His His His His
   180 185 190
   His His
<210> 17
   <211> 148
   <212> PRT
   <213> Artificial Sequence
<220>
   <223> Em95-His
<400> 17
   Gln Glu Tyr Arg Gly Arg Gly Ile Glu Ile Lys Thr Thr Glu Ser Pro
   1               5                   10                  15
   Leu Arg Lys His Phe Ser Leu Thr Leu Val Gly Ser Gln Gly Ile Arg
               20                  25                  30
   Leu Ser Trp Asp Val Gln His Leu Pro Asp Leu Arg Gly Thr Asn Ile
           35                  40                  45
   Ser Leu Lys Ala Leu Asp Pro Ser Asp Pro Leu Val Tyr Lys Arg Gln
       50                  55                  60
   Thr Ala Gln Phe Ser Asp Gly Gln Leu Ala Ile Gly Arg Leu Lys Pro
   65                  70                  75                  80
   Ser Thr Leu Tyr Gln Met Thr Val Glu Ala Val Arg Gly Lys Asn Thr
                   85                  90                  95
   Thr Leu Lys Phe Thr Glu Asp Ile Lys Thr Leu Arg Ile Gly Glu Lys
               100                 105                 110
   Glu Ser Thr Val Met Thr Ser Gly Ser Ala Leu Thr Ser Ala Ile Ala
           115                 120                 125
   Gly Phe Val Phe Ser Cys Ile Val Ile Val Leu Thr Leu Glu His His
       130                 135                 140
   His His His His
   145

## Description

The present invention refers to a diagnostically useful carrier comprising a means for specifically capturing an antibody to p21 from *Echinococcus multilocularis* in a sample from a subject; a kit comprising the diagnostically useful carrier, preferably further comprising a means for detecting any captured antibody; a method comprising the step detecting the presence or absence of an antibody to p21 from *Echinococcus multilocularis* in a sample from a subject; a use of p21 from *Echinococcus multilocularis* or a variant thereof for increasing the diagnostic reliability, preferably the sensitivity of an assay for the detection of an *Echinococcus* infection, preferably for distinguishing an *Echinococcus multilocularis* infection from an *Echinococcus granulosus* infection; a polypeptide running at a molecular weight of 21 kDa from *Echinococcus multilocularis,* obtainable by separating according to molecular weight an extract from *Echinococcus multilocularis* using gel electrophoresis, or a variant thereof, or an antibody to said polypeptide.

Helminth infection or worm infection, also known as Helminthiasis, is a macroparasitic disease transmittable to mammals such as humans, dogs, foxes and wolves, characterized by infection of the body with helminths such as tapeworms of the *Echinococcus* type. *E. granulosus and E. multilocularis* are two of the world's major parasites. More than one million people are infected with one of them at any one time.

There are two main types of *Echinococcus* infections, cystic echinococcosis and alveolar echinococcosis. Human infections occur following ingestion of eggs. Larvae are released in the host gut, penetrate intestinal villi and migrate to various organs.

The disease often starts without symptoms and this may last for a year. The symptoms and signs that occur subsequently depend on the cyst's location and size. Alveolar disease usually begins in the liver but can spread to other parts of the body such as the lungs or brain. When the liver is affected the person may suffer from abdominal pain as well as weight loss and turn yellow. Lung disease may cause pain in the chest, shortness of breath and coughing. Without treatment, *Echinococcus* infections may be lethal.

Since the patient may be asymptotic for some time and it becomes more and more difficult to provide effective treatment as the disease progresses, reliable tools for the early serodiagnosis of human helminthic infections are highly sought-after.

Patients infected with *Echinococcus* display a variety of immune responses (Gottstein, B., Mesarina, B., Tanner, I., Ammann, R. W., Wilson, j. F., Eckert, J. Lanier, A. (1991): Specific Cellular and Humoral Immune Responses in Patients with Different Long-Term Courses of Alveolar Echinococcosis (Infection with Echinococcus multilocularis). Am. J. Trop Med. Hy. 45, 734-742.

Therefore, conventional immunoassays based on native antigens, which may be extracted from whole worms or *in vitro* cultures, may be used in immunoblots to detect host antibodies to the parasite (Ayadi, A., Dutoit, B., Sendid, B., and Camus, D (1995): Specific Diagnostic Antigens of Echinococcus granulosus detected by Western Blot. Parasite 2, 119-123). Such assays are commercially available, for example from EUROIMMUN, Lübeck (Anti-Echinococcus-granulosus WESTERNBLOT).

These natural antigens are limited in availability, suffer from batch-to-batch variation and lack the degree of diagnostic reliability that would be desirable. Further shortcomings include the fact that they are often cross-reactive, *i.e.* recognized by sera from patients infected with two or more helminth species.

In addition to detecting *Echinococcus,* parasitologists have an interest in distinguishing material from *E. granulosus* and *E. multilocularis.* Although the larvae of both parasites are predominantly found in the liver, their distribtions throughout the host's body differs at later developmental stages, with significant implications for the treatment of patients infected. For example, *E. granulosus* is most frequently found in the lung, whilst *E. multilocularis* may enter both the lung and the brain of the host.

Therefore, a problem underlying the present invention is to provide an assay for or aiding in the detection of *Echinococcus* infections which is improved compared to state of the art assays with regard to one or more of their various shortcomings.

Another problem underlying the present invention is to provide an assay for the detection of *Echinococcus* having a higher degree of reliability, in terms of specificity and/or sensitivity, with regard to the detection of *Echinococcus multilocularis* and *Echinococcus granulosus* infections compared to state of the art assays.

Another problem underlying the present invention is to provide an assay having a higher degree of reliability with regard to the distinction between *Echinococcus multilocularis* and *Echinococcus granulosus* infections compared to state of the art assays.

The problem underlying the present invention is solved by the subject-matter of the attached independent and dependent claims.

In a first aspect, the problem underlying the present invention is solved by a diagnostically useful carrier comprising a means for specifically capturing an antibody to p21 from *Echinococcus multilocularis* in a sample from a subject.

The carrier further comprises a means for specifically capturing an antibody to one or more polypeptides selected from the group comprising p7 and p16/18 and optionally p25/26 from *Echinococcus multilocularis,* preferably all of them.

In another preferred embodiment of the first aspect, the means for specifically capturing an antibody to a polypeptide from the group comprising p21, p7, p16/18 and p25/26 from *Echinococcus multilocularis* is the respective polypeptide from the group comprising p21, p7, p16/18 and p25/26 from *Echinococcus multilocularis* or a variant thereof, preferably in a native form of the polypeptide.

In another preferred embodiment of the first aspect, the means for specifically capturing an antibody to a polypeptide from the group comprising p21, p7, p16/18 and p25/26 from *Echinococcus multilocularis* is provided in the form of a polypeptide extract from *Echinococcus,* preferably *Echinococcus multilocularis,* more preferably separated according to molecular weight,
and, and wherein the polypeptide extract comprises p16/18 and p21 from *Echinococcus multilocularis* which are separated sufficiently to allow for distinction between an antibody to p16/18 and another antibody to p21. In particular, the distance between p16/18 and p21 on the carrier is such that the bands associated with them do not overlap to such an extent that a signal indicating binding of an antibody to p16/18 cannot be distinguished from a signal indicating binding of an antibody to p21. In other words, the carrier may be used to detect whether antibodies to p16/18, to p21 or antibodies to both p16/18 and p21 are present in the sample.

The carrier further comprises a means for specifically capturing an antibody to one or more polypeptides selected from the group comprising AgB8/1a, AgB8/1b, AgB8/2, AgB8/3, AgB8/4a and AgB8/4b from *Echinococcus granulosus,* Em18 and Em95 from *Echinococcus multilocularis,* preferably all of them.

In another preferred embodiment of the first aspect, the means for specifically capturing an antibody to one or more polypeptides selected from the group comprising AgB8/1a, AgB8/1b, AgB8/2, AgB8/3, AgB8/4a and AgB8/4b from *Echinococcus granulosus,* Em18 and Em95 from *Echinococcus multilocularis* is the respective polypeptide from the group comprising AgB8/1a, AgB8/1b, AgB8/2, AgB8/3, AgB8/4a and AgB8/4b from *Echinococcus granulosus,* Em18 and Em95 from *Echinococcus multilocularis* or a variant thereof, preferably provided in the form of one or more recombinant and/or purified polypeptides.

In a second aspect, the problem underlying the present invention is solved by a kit comprising the diagnostically useful carrier according to the present invention, preferably further comprising a means for detecting any captured antibody.

In a third aspect, the problem underlying the present invention is solved by a method comprising the step detecting an antibody to p21 from *Echinococcus multilocularis* in a sample from a subject.

The method further comprises detecting an antibody to one or more polypeptides selected from the group comprising p7 and p16/18 and optionally p25/26, preferably all of them, more preferably p7 and p16/p18, most preferably p7.

The method further comprises the step detecting an antibody to one or more polypeptides selected from the group comprising AgB8/1a, AgB8/1b, AgB8/2, AgB8/3, AgB8/4a and AgB8/4b from *Echinococcus granulosus,* Em18 and Em95 from *Echinococcus multilocularis,* preferably all of them.

In another preferred embodiment of the third aspect, antibodies to more than one of the polypeptides from said group are detected simultaneously.

In another preferred embodiment of the third aspect, the antibodies to more than one of the polypeptides from said group are detected in spatially separate binding reactions or in a one-pot reaction, preferably in a one-pot reaction.

In another preferred embodiment of the third aspect, wherein an antibody to p21 detected can be distinguished from an antibody binding to p16/18.

Also disclosed is the use of p21 from *Echinococcus,* preferably *Echinococcus multilocularis* or a variant thereof for increasing the diagnostic reliability, preferably the sensitivity of an assay for the detection of an *Echinococcus* infection, more preferably for distinguishing an *Echinococcus multilocularis* infection from an *Echinococcus granulosus* infection.

Disclosed herein is a polypeptide having a molecular weight of 21 kDa from *Echinococcus multilocularis or a variant thereof,* obtainable by separating according to molecular weight an extract from *Echinococcus multilocularis* using gel electrophoresis or an antibody to said polypeptide.

The present invention is based on the inventors' surprising finding that a novel *Echinococcus* polypeptide, referred to throughout this application as "p21", exists that, when incorporated in assays for the detection of an *Echinococcus* infection, may enhance the overall assay sensitivity.

The present invention centers around an assay for the diagnosis an *Echinococcus* infection that involves detecting in a sample from a subject suspected of having an *Echinococcus* infection antibodies to polypeptide p21 from *Echinococcus multilocularis.* p21 is a polypeptide having an apparent molecular weight of approximately 21 kDa as judged by SDS PAGE under reducing conditions and comparison with the running behavior of standard molecular weight marker proteins running under the same conditions. The p21 polypeptide band runs between the bands of the p16/p18 and p25/26 polypeptides described in the state of the art. Detection of antibodies to p21, and preferably to one or more polypeptides from the group comprising p7, p16/18 and p25/26 from *Echinococcus multilocularis,* AgB8/1a, AgB8/1b, AgB8/2, AgB8/3, AgB8/4a and AgB8/4b from *Echinococcus granulosus* and Em18 and Em95 from *Echinococcus multilocularis* may corroborate the assumption that the patient suffers from an *Echinococcus* infection and provide diagnostically relevant information. Preferably the diagnostically useful carrier comprises p16/18 and p21 from *Echinococcus multilocularis* or a variant thereof, which are separated sufficiently to allow for distinction between an antibody to p16/18 and another antibody to p21. More preferably, it may comprises in addition, in order of increasing preference, p7; p7 and Em18 from *Echinococcus multilocularis;* p7, Em18 and Em95 from *Echinococcus multilocularis;* or p7, Em18 and Em95 from *Echinococcus multilocularis* and AgB8/1a, AgB8/1b, AgB8/2, AgB8/3, AgB8/4a and AgB8/4b from *Echinococcus granulosus* or variants thereof. Preferably any polypeptide from the group comprising p7, p16/18 and p21 is a native polypeptide, whereas any polypeptide from the group comprising Em18 and Em95 from *Echinococcus multilocularis* and AgB8/1a, AgB8/1b, AgB8/2, AgB8/3, AgB8/4a and AgB8/4b from *Echinococcus granulosus is recombinant.* Such a carrier may be used to detect an *Echinococcus* infection at higher sensitivity.

The native protein is obtainable by preparing a polypeptide extract from *Echinococcus multilocularis* preferably following the protocol by Spiliotis, M., Tappe, D., Sesterhenn, L., and Brehm, K. (2004) Long-termin in vitro cultivation of Echinococcus multilocularis metacestodes under axenic conditionsParasitol. Res. 92 (5), 430-432. p21 may be detected, throughout the purification process, by way of immunoblotting, using a sample from an infected patient reactive with p21 as an antibody source. Preferably p21 is enriched relative to its proportion to other polypeptides in native tissue, for example by separating the polypeptide extract according to size, collecting fractions and preparing a mixture comprising a volume of p21-containing fractions that exceeds the volume of other fractions. Preferably p21 used according to the invention is non-recombinant p21 purified from a tissue.

The protein band comprising p21 may be cut out from the gel following completion of the electrophoresis and the protein eluted using standard protein purification techniques. It may be separated from any polypeptide running at a similar molecular weight by way of affinity chromatography, using a sample from an infected patient reactive with p21 to obtain the antibody to p21 as the affinity ligand. The person skilled in the art is familiar with such methods.

Alternatively, a piece of electrophoresis gel comprising polypeptides from *Echinococcus,* preferably *Echinococcus multilocularis* having an enriched proportion of polypeptides, among them p21, having a certain range of molecular weights as judged by standard molecular weight markers, compared to a non-processed polypeptide extract, may be cut out from an electrophoresis gel obtained by subjecting a polypeptide extract from *Echinococcus,* preferably *Echinococcus multilocularis* to preparative gel electrophoresis. Preferably, the molecular weight range on the gel includes polypeptides from 1 to 200 kDa, more preferably 5 to 100 kDa, more preferably 6 to 50 kDa and more preferably 7 to 30 kDa, wherein, most preferably, native *Echinococcus,* preferably *Echinococcus multilocularis* polypeptides outside said molecular weight range are present at a lower proportion than in the polypeptide extract obtained directly from the organism. Polypeptides outside said molecular weight range may optionally be absent, for example as a result of cutting out the gel piece comprising the sought-after molecular weight range and discarding the remainder of the gel. Other options are available for producing a fraction of *Echinococcus,* preferably *Echinococcus multilocularis* polypeptides enriched in those polypeptides, compared to a non-processed polypeptide extract, within said molecular weight range, for example the use of size-exclusion chromatography.

It is advantageous that any separation according to molecular weight is sufficient to allow for distinction between p21 and any other polypeptide having a similar molecular weight, for example p18 and p25/p26. The person skilled in the art is familiar with suitable techniques, for example gel electrophoresis using gels with a polyacrylamide concentration tailored specifically for the efficient separation of polypeptides having a certain molecular weight, for example 21 kDa. The amount of p21, for any aspect of the present invention, needs to be sufficient for detection separable from any background signals, for example for detection using a secondary labeled antibody, wherein preferably the label has enzymatic activity or is chemiluminescent, fluorescent or radioactive.

The *Echinococcus multilocularis* polypeptides having said molecular weight range, optionally, associated with said piece of electrophoresis gel, may be transferred, for example by electroblotting, to a diagnostically useful, preferably solid carrier, optionally in the shape of a test strip, preferably a line blot. The solid carrier may comprise one or more recombinant and/or purified diagnostically relevant polypeptides from *Echinococcus multilocularis* in addition. In a preferred embodiment, the term "purified" polypeptide, as used herein, refers to a polypeptide that constitutes at least 50, 60, 70, 80, 90, 95 or 99 % of all polypeptides present as judged by SDS PAGE following Coomassie staining and visual inspection.

The invention relates to a diagnostically useful carrier, which is preferably a solid carrier for contacting a means for specifically capturing an antibody, which means is associated with said carrier, with a bodily fluid sample from a subject, preferably a human subject. In a preferred embodiment the solid carrier is a diagnostic device, more preferably selected from the group comprising one or more beads, a test strip, a microtiter plate, a blot, a glass surface, a biochip and a membrane, more preferably from the group comprising a blot, a test strip and a membrane. In a most preferred embodiment, the diagnostically useful carrier is a line blot (Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540). In a preferred embodiment, the term "line blot", as used herein, refers to a test strip, more preferably membrane-based, that has been coated with one or more means for capturing an antibody, preferably a polypeptide each. If two or more means are used, they are preferably spatially separated on the carrier, preferably along the longitudinal axis of a carrier shape in the form of a test strip. Preferably, the width of the bands is at least 30, more preferably 40, 50, 60, 70 or 80 % the width of the test strip. The test strip may comprise one or more control bands for confirming that it has been contacted with sample sufficiently long and under adequate conditions, in particular in the presence of human serum, antibody conjugate, or both. A multitude of diagnostic solutions including line blots are commercially available, for example from EUROIMMUN, Lübeck, Germany.

It is essential that the sample used to practice the present invention comprises antibodies, also referred to as immunoglobulins. Typically the sample is a bodily fluid comprising a representative set of the subject's antibodies. The sample may be selected from the group comprising whole-blood, serum, cerebrospinal fluid and saliva and is preferably serum or plasma, more preferably serum. Preferably the antibodies are human antibodies.

It is essential that the diagnostically useful carrier comprises a means for specifically capturing an antibody to one or more antigenic polypeptides of diagnostic relevance from *Echinococcus,* preferably *Echinococcus multilocularis.* The diagnostically useful carrier according to the invention serves as a scaffold for the one or more of these means, which are molecule capturing specifically the antibody of interest. The person skilled in the art is aware of a multitude of methods to make and identify molecules with desirable binding properties or to tailor existing molecule. These molecules include, but are not limited to polypeptides, aptamers, small molecules and derivatives thereof, and are preferably antigenic polypeptides. Said carrier is suitable for carrying out a diagnostic method.

One first group of antigenic polypeptides of diagnostic relevance includes, in addition to p21, the polypeptides of the group comprising p7, p16/18 and p25/26 from *Echinococcus multilocularis.* These are known antigenic polypeptides, preferably non-recombinant, named in line with their molecular weight (Asian, M., Yüksel, P, Polat, E., Cakan, H., Ergin, S., Öner, Y. A., Zengin, K., Arikan, S., Saribas, A., Mamal Torum, M., Kocazeybek, B. (2011) The diagnostic value of Western blot method in patients with cystic echinococcosis, New. Microbiol. 23, 173-177). A second group of antigenic polypeptides of diagnostic relevance includes those from the group comprising AgB8/1a, AgB8/1b, AgB8/2, AgB8/3, AgB8/4a and AgB8/4b from *Echinococcus granulosus,* Em18 and Em95 from *Echinococcus multilocularis,* wherein the terms "AgB8/1a", "AgB8/1b", "AgB8/2", "AgB8/3", "AgB8/4a" and "AgB8/4b" from *Echinococcus granulosus,* "Em18" and "Em95" from *Echinococcus multilocularis,* refer to the sequences represented by data base codes SEQ ID NO1 (Eg AgB8/1a), SEQ ID NO 2 (EG AgB8/1b), SEQ ID NO 3 (Eg AgB8/2), SEQ ID NO 4 (Eg AgB8/3), SEQ ID NO 5 (Eg AgB8/4a), SEQ ID NO 6 (Eg_AgB8/4b), SEQ ID NO 7 (*Echinococcus multilocularis* Em18) and SEQ ID No 8 (*Echinococcus multilocularis* Em95), respectively. Optionally one or more of these polypeptides or variants thereof, for example those from the group comprising AgB8/1a, AgB8/1b, AgB8/2, AgB8/3, AgB8/4a and AgB8/4b from *Echinococcus granulosus,* may be fused to generate a fusion protein such as SEQ ID NO 9. Any data base codes referred to throughout this application refer to the respective polypeptide sequence available via the NCBI data bases as online at the priority date of this application.

According to the present invention, the carrier comprises one or more means for specifically capturing an antibody, preferably one or more, more preferably two or more, more preferably three or more, more preferably four or more such means, each of them capable of specifically capturing a different antibody, as defined in present claims. Said means is preferably immobilized on said carrier. In a preferred embodiment, the means for specifically capturing an antibody of interest is an antigenic polypeptide comprising or consisting of the antigenic polypeptide to which the antibody binds, preferably from the group comprising p21, p7, p16/18 and p25/26 from *Echinococcus multilocularis,* AgB8/1a, AgB8/1b, AgB8/2, AgB8/3, AgB8/4a and AgB8/4b from *Echinococcus granulosus* and Em18 and Em95 from *Echinococcus multilocularis.* Said antigenic polypeptide contains one or more characteristic epitopes comprising at least 7, preferably 10 more preferably 15 amino acids which confer the ability to bind specifically to the antibody in a sample from an infected patient. Said antigenic polypeptide, together with the insoluble carrier to which it is attached, may be separated from a reaction mixture comprising a sample to be analyzed in a straightforward manner, for example by filtration, centrifugation or decanting. One or more of these antigenic polypeptides, for example p21 from *Echinococcus multilocularis,* may be used to manufacture a medicament for treatment of a disease or used for the manufacture of an agent for diagnosis of disease a disease, which disease is an *Echinococcus* infection.

Said antigenic polypeptide may be immobilized in a reversible or irreversible manner. For example, the immobilization is reversible if the molecule interacts with the carrier via ionic interactions which may be masked by addition of a high concentration of salt or if the molecule is bound via a cleavable covalent bond. By contrast, the immobilization is irreversible if the molecule is linked to the carrier via a covalent bond that cannot be cleaved in aqueous solution. The polypeptide may be indirectly immobilized, for example by immobilizing an antibody or other entity having affinity to the polypeptide, followed by addition of the polypeptide and formation of a polypeptide-antibody complex.

However, the teachings of the present invention may not only be carried out using polypeptides having the exact sequences referred to in this application explicitly, for example by function, name, sequence or accession number, or implicitly, but also using variants of such polypeptides.

In a preferred embodiment, the term "variant", as used herein, may refer to at least one fragment of the full length sequence referred to, more specifically one or more amino acid or nucleic acid sequence which is, relative to the full-length sequence, truncated at one or both termini by one or more amino acids. Such a fragment comprises or encodes for a peptide having at least 10, 15, 25, 50, 75, 100, 150 or 200 successive amino acids of the original sequence or a variant thereof. The total length of the variant may be at 25, 30, 40, 50, 60, 70, 80, 90, 100 or more amino acids. In the case of a native protein, such fragments may be made by limited proteolysis. In the case of a recombinant protein, an additional possibility is to express truncated polypeptides.

In another preferred embodiment, the term "variant" relates not only to at least one fragment, but also a polypeptide or a fragment thereof comprising amino acid sequences, preferably a fragment comprising at least 25, more preferably 50, more preferably 200 successive amino acids, that are at least 40, 50, 60, 70, 75, 80, 85, 90, 92, 94, 95, 96, 97, 98 or 99 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. The state of the art comprises various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings.

In a preferred embodiment, variants may, in addition, comprise chemical modifications, for example isotopic labels or covalent modifications such as glycosylation, phosphorylation, acetylation, decarboxylation, citrullination, hydroxylation and the like. This may include the chemical, non-recombinant ligation or a native or recombinant protein with a native, recombinant or chemically synthesized protein. The person skilled in the art is familiar with methods for the modification of polypeptides. Moreover, variants may also be generated by way of fusion with other known polypeptides or variants thereof.

The variant of the polypeptide has biological activity. In a preferred embodiment such biological activity is the ability to capture an antibody to the respective polypeptide from a sample obtained from a subject infected with *Echinococcus* that comprises such an antibody, but not from a sample obtained from a healthy subject.

Any polypeptide used to carry out the inventive teachings, including any variants, is designed such that it antigenic, i.e. comprises epitopes recognized by specifically by antibodies from patients suffering from an *Echinococcus* infection (but not by antibodies from healthy subjects), more preferably from an *E. granulosus* or *E. multilocularis* infection. In one embodiment, such polypeptide comprises a stretch of 6, 7, 8, 9, 10, 11, 12, 20, 25, 30, 40, 50, 60, 70, 80, 90, 100 or more, preferably at least 9 but no more than 16, consecutive amino acids from the respective native *Echinococcus* polypeptide. The person skilled in the art is familiar with guidelines used to design peptides having sufficient immunogenicity, for example those described in Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173. Briefly, it is desirable that the peptide meets as many as possible of the following requirements: (a) it has a high degree of hydrophilicity, (b) it comprises one or more residues selected from the group comprising aspartate, proline, tyrosine and phenylalanine, (c) is has, for higher specificity, no or little homology with other known peptides or polypeptides, (d) it needs to be sufficiently soluble and (e) it comprises no glycosylation or phosphorylation sites unless required for specific reasons. Alternatively, bioinformatics approaches may be followed, for example those described by Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71.

If an antigenic polypeptide is used as the means for specifically capturing an antibody, for example to p21, said polypeptide, when used to carry out the teachings of the present invention, may be provided in any form and at any degree of purification, from tissues or cells comprising said polypeptide in an endogenous form, more preferably cells overexpressing the polypeptide, crude or enriched lysates of such cells, to purified and/or isolated polypeptide which is essentially pure. In a preferred embodiment, the polypeptide is a native polypeptide, wherein the term "native polypeptide", as used herein, refers to a folded polypeptide, more preferably to a folded polypeptide purified from tissues or cells, more preferably from mammalian cells or tissues, optionally from non-recombinant tissues or cell. If a native polypeptide is used, it is preferably enriched compared to its natural state. The antibody captured or detected according to the present invention is preferably an IgG class antibody.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification). In another preferred embodiment, the polypeptide is an isolated polypeptide, wherein the term "isolated" means that the polypeptide has been enriched compared to its state upon production using a biotechnological or synthetic approach and is preferably pure, i.e. at least 60, 70, 80, 90, 95 or 99 percent of the polypeptide in the respective sample consists of said polypeptide as judged by SDS polyacrylamide gel electrophoresis followed by Coomassie blue staining and visual inspection.

The subject according to the present invention is an organism producing antibodies, preferably infection-related antibodies, more preferably from a mammal, most preferably a human. Preferably none of the methods according to the present inventions are practiced on the human or animal body, but under *in vitro* conditions.

Within the scope of the present invention is a diagnostically useful carrier comprising a means for specifically capturing an antibody to an antigenic polypeptide such as p21. In a preferred embodiment, the term "specifically capturing an antigen", as used herein, refers to the ability to bind specifically to the antibody of interest to the effect that it is bound and removed from the sample rather than binding to any antibody or any antibody from a certain antibody class present in the sample.

According to the present invention, a means for specifically detecting a captured antibody is provided, optionally as part of a kit. In a preferred embodiment, the term "specifically detecting a captured antibody", as used herein, means that the antibody binding specifically to the means for specifically capturing the antibody, preferably p21, following capture, is detected rather than the entirety of the antibodies present in the sample due to the ability of the means to bind specifically to the variable region of the antibody determining the antibody's binding specificity rather than its constant region. In a preferred embodiment, the term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25 °C in PBS buffer at pH 7.

In a preferred embodiment, the means for specifically capturing an antibody to a first antigenic polypeptide according to the present invention and the means for specifically capturing an antibody to a second antigenic polypeptide according to the present invention are on separate carriers. This means that the means are not attached to a single carrier, but one or more carriers that are separate and/or separable without damaging them. For example, the means for specifically capturing an antibody p21 may be attached to a first test strip, and the detecting an antibody to p16 is attached to another test strip which is separate from the first test strip.

In another preferred embodiment, the means for specifically capturing an antibody to a first antigenic polypeptide according to the present invention and the means for specifically capturing an antibody to a second antigenic polypeptide according to the present invention are on one, preferably covalently linked to one carrier. This means that the means are attached to one carrier which cannot be disassembled, without damaging the carrier, such that the means are on separate carriers. For example, the means may be all coated on one test strip, particular in the form of a line blot.

The teachings according to the present invention provide a kit, preferably for diagnosing an *Echinococcus* infection, more preferably for distinguishing between an *E. granulosus* from an *E multilocularis* infection. Such a kit is a container that comprises specific reagents required to practice the method according to the present invention, in particular the diagnostically useful carrier according to the present invention, optionally in addition to one or more solutions required to practice the method according to the present invention, preferably selected from or all from the group comprising sample dilution buffer, washing buffer and buffer comprising a means for detecting any specifically captured antibody, such as a secondary antibody and optionally a means for detecting the latter. Furthermore, it may comprise instructions detailing how to use the kit and the diagnostically useful carrier according to the present invention for contacting the polypeptide according to the present invention with a bodily fluid sample from a subject, preferably a human subject, for example a line blot, wherein the means for specifically capturing p21 according to the present invention, preferably a polypeptide comprising p21 or a variant thereof, is immobilized on the line blot. Furthermore, the kit may comprise a positive control, for example an antibody known to bind to p21, and a negative control, for example a protein having no detectable affinity to p21 such as bovine serum albumin. Finally, such a kit may comprise a standard solution comprising a p21-binding antibody for preparing a calibration curve. In a preferred embodiment, the kit comprises a device, preferably a blot-based device such as a line blot coated with a means for specifically capturing an antigenic polypeptide according to the present invention.

According to the invention, a means for detecting the one or more captured antibodies may be provided. The person skilled in the art is aware of many methods that may be used. In a preferred embodiment, a secondary antibody binding to the constant region of the one or more captured antibodies, which is the corresponding primary antibody, is used, which secondary antibody may be associated with a label that is straightforward to detect, for example a fluorescent, radioactive or enzymatically active label, the latter of which may catalyze a chemiluminescent reaction or the generation of a molecule detectable using colourimetry or spectroscopy or another analytical method.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from certain a disease or disorder in the past, at the time of the diagnosis or in the future, to find out how the disease is progressing or is likely to progress in the future or to evaluate the responsiveness of a patient with regard to a certain treatment, for example the administration of suitable drugs. In other words, the term "diagnosis" comprises not only aiding the diagnosis, but also a prognosis and/or efforts to monitor the course of a disease or disorder.

Therefore, the term "diagnosis" does preferably not imply that the diagnostic methods or agents according to the present invention will be definitive and sufficient to finalize the diagnosis on the basis of a single test, let alone parameter. By contrast it has to be emphasized that a plethora of further information must be gathered, in particular by imaging techniques (ultrasound scanning and computerized tomography) and must be weighed carefully by a medical doctor to obtain a coherent picture which allows said doctor to provide a final diagnosis.

The term "diagnosis" may refer to a contribution to what is referred to as a "differential diagnosis", *i*. *e.* a systematic diagnostic procedure considering the likelihood of a range of possible conditions on the basis of a range of diagnostic parameters. The term "diagnosis" may also refer to a method or agent used to choose the most promising treatment regime for a patient. In other words, the method or agent may relate to selecting a treatment regimen for a subject.

The present invention relates to a method comprising the step detecting in a sample from a subject an antibody to an antigenic polypeptide such as p21. Such a method may comprise the steps a) providing in a sample from a subject, b) contacting the sample with the diagnostically useful carrier according to the present invention under conditions compatible with the formation of a complex comprising the diagnostically useful carrier and the antibody, more specifically the means for specifically capturing the antibody bound to the antigen, c) isolating any said complex, for example by removing the sample, d) optionally washing said complex, and e) detecting said complex. The method is preferably an *in vitro* method. The detection of an antibody, more specifically said complex according to the present invention comprises the use of a method selected from the group comprising immunodiffusion techniques, immunoelectrophoretic techniques, light scattering immunoassays, light scattering immunoassays, agglutination techniques, labeled immunoassays such as those from the group comprising radiolabeled immunoassay, enzyme immunoassays such as colourimetric assays, chemiluminscence immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14.

In many cases detecting an antibody, optionally meaning determining whether the concentration of the antibody in the sample is beyond a certain threshold is sufficient for the diagnosis. If the antibody can be detected, this result will be instrumental for the clinician's diagnosis and indicates an increased likelihood that the patient suffers from a disease. In a preferred embodiment, the relative concentration of the antibody in the serum, compared to the level that may be found in the average healthy subject, may be determined. In a preferred embodiment, the term "detecting", as used herein, means that it is sufficient to check whether a signal sufficiently beyond any background level may be detected using a suitable complex detection method that indicates that the antibody of interest is present or more antibody of interest is present than would be in a healthy subject. In a more preferred embodiment this may involve determining whether the concentration is at least 0.1, preferably 0.2, 0.5, 1, 2, 5, 10, 20, 25, 50, 100, 200, 500, 1000, 10000 or 100000 times higher than the concentration of the antibody of interest found in the average healthy subject.

In a preferred embodiment, at least two antibodies, each to a different antigenic polypeptide according to the present invention, are detected simultaneously, *i.e.* at the same time. This is convenient in terms of efficient diagnostic procedures, as a maximum of diagnostic information is obtained in a given period of time. Of course a prerequisite is that sufficient capacity is available for running all reactions.

In a preferred embodiment, at least two antibodies, each to an antigenic polypeptide according to the present invention, are detected in spatially separate reactions. This means that these reactions are run in different reaction mixtures in separate vessels.

If more than one antibody is to be detected, the method may be carried out in a one-pot reaction. Preferably, the term "one-pot reaction", as used herein, means that two or more, preferably all reactions carried out for the purpose of detecting the presence or absence of an antibody are carried out in the same reaction mixture in one reaction vessel, without physical barriers between the reactions, by contrast to experimental settings contemplating that at least two reactions are carried out in separate solutions and reaction vessels. The one-pot mode is advantageous, since only one reaction has to be set up, thus saving time and resources. The minimum volume of sample required may be reduced.

The present invention is further illustrated by the following figures and non-limiting examples from which further features, embodiments, aspects and advantages of the present invention may be taken.
**Fig. 1** shows a diagnostically useful carrier according to the present invention. It is a line blot comprising a section coated with *E. multilocularis* polypeptide extract polypeptides in the molecular weight range from 7 to 160 kDa, separated according to molecular weight prior by way of electrophoresis following by transfer onto the line blot by electroblotting.
   In addition it comprises a recombinant fusion protein AgB8/1a, AgB8/1b, AgB8/2, AgB8/3, AgB8/4a and AgB8/4b from *Echinococcus granulosus* and recombinant Em18 and Em95 from *Echinococcus multilocularis.*

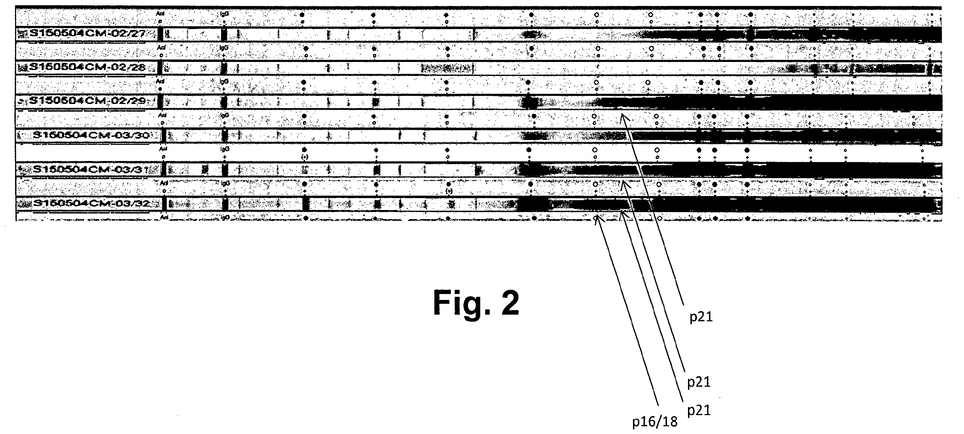

**Fig. 2** shows a range of line blots produced in the same manner as described before. Each has been contacted with a different patient sample. Comparison of the various developed blots shows that p21 is an antigenic polypeptide distinct from previously used antigenic polypeptides such as p18.
**Fig. 3** shows the part of line blots that comprises the native *Echinococcus* proteins separated according to molecular weights.

A number of polypeptide sequences are referred to throughout this application. They include:

| *SEQ ID NO* | *Polypeptide (E.g.* = *E. granulosus; E. m.* = *E. multilocularis)* |
|---|---|
| *SEQ ID NO 1* | *Eg AgB8*/*1a* |
| *SEQ ID NO 2* | *Eg AgB8*/*1b* |
| *SEQ ID NO 3* | *Eg AgB8*/*2* |
| *SEQ ID NO 4* | *Eg AgB8*/*3* |
| *SEQ ID NO 5* | *Eg AgB8*/*4a* |
| *SEQ ID NO 6* | *Eg AgB8*/*4b* |
| *SEQ ID NO 7* | *Em18* |
| *SEQ ID NO 8* | *Em95* |
| *SEQ ID NO 9* | *Fusion comprising Eg AgB8*/*1a, Eg AgB8*/*1b, Eg AgB8*/*2, Eg AgB8*/*3, Eg AgB814a, Eg AgB8*/*4b* |
| *SEQ ID NO 10* | *His-GST fusion comprising Eg AgB8*/*1a* |
| *SEQ ID NO 11* | *His-GST fusion comprising Eg AgB8*/*1b* |
| *SEQ ID NO 12* | *His-GST fusion comprising Eg AgB8*/*2* |
| *SEQ ID NO 13* | *His-GST fusion comprising Eg AgB8*/*3* |
| *SEQ ID NO 14* | *His-GST fusion comprising Eg AgB8*/*4a* |
| *SEQ ID NO 15* | *His-GST fusion comprising Eg AgB8*/*4b* |
| *SEQ ID NO 16* | *His fusion comprising Em18* |
| *SEQ ID NO 17* | *His fusion comprising Em95* |

### Example 1: Production of the diagnostically useful carrier

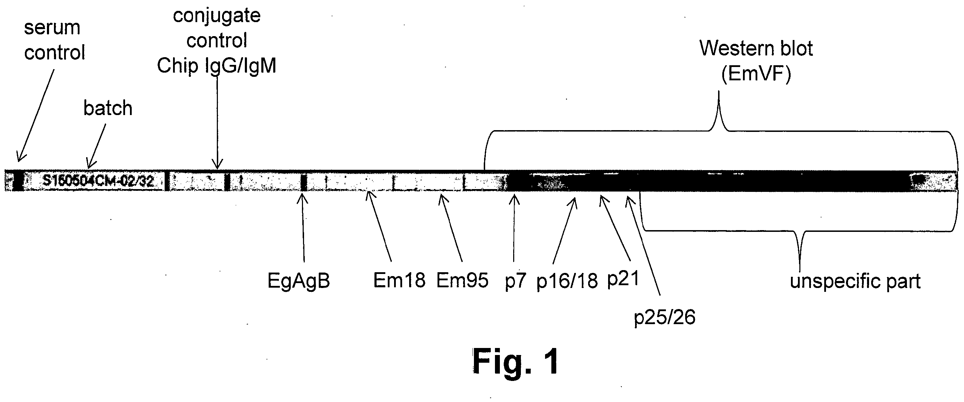

The line blot depicted in **Fig. 1** was used for the experimental programme described herein. It was prepared by electrophoretically separating polypeptides according to molecular weight from *E. multilocularis* polypeptide extract comprising proteins p7, p16/18, p21 and p25/26 in the molecular weight range from 7 to 160 kDa, as prepared as described in Spiliotis, M., Tappe, D., Sesterhenn, L., and Brehm, K. (2004) Long-termin in vitro cultivation of Echinococcus multilocularis metacestodes under axenic conditions Parasitol. Res. 92 (5), 430-432., with minor modifications, and transferring the resulting separated polypeptides on a membrane, wherein the pattern of polypeptides as separated remains intact. In addition, the membrane was coated using recombinant fusion protein comprising AgB8/1a, AgB8/1b, AgB8/2, AgB8/3, AgB8/4a and AgB8/4b (SEQ ID NO 9) and recombinant polypeptides Em18 (SEQ ID NO 16) and Em95 (SEQ ID NO 17). All recombinant proteins had been purified using standard protocols for the isolation of GST and/or His tagged protein.

### Example 2: Evaluation of the diagnostic reliability

To determine sensitivity, specificity, species differentiation rate and cross reactivity to other parasite infection a study was performed using 50 sera obtained from healthy blood donors, 50 sera obtained from tumor patients, 52 sera obtained from patients infected with *E. multilocularis,* 55 sera obtained from patients infected with *E. granulosus,* 122 sera obtained from patients infected with other parasites (n=5 *Filaria,* n=10 Toxocara, n=16 Anisakis, n=10 Strongyloides, n=9 Schistosoma, n=2 Multi-Helminths, n=7 Plasmodium, n=10 Ascaris, n=7 Taenia, n=13 Trichinella, n=17 Fasciola, n=11 Entamoeba, n=5 Leishmania). Patients were examined comprehensively by an expert clinician to establish the respective diagnosis. This included diagnostic examinations based on serology, sonography and computer tomography.

The blot produced as described in Example 1 was subsequently contacted with patient sera and developed using the reagents and instructions supplied with the commercially Anti-*Echinococcus granulosus* Westernblot (DY2320-G, EUROIMMUN, Lübeck).

If antibodies to at least one of the antigens from the group comprising Em18, Em95, the AgB fusion protein, p7, p16/18 and p21 could be detected, it was concluded that the respective patient suffers from an *Echinococcus* infection.

Antibodies to Em18 and/or Em95 were taken to indicate an *Echinocuccus multilocularis* infection, whereas antibodies to the AgB fusion protein in the absence of antibodies to Em18 and/or Em95 were taken to indicate an *Echinocuccus granulosus* infection.

### Results:

The study revealed a sensitivity of 93%, a specificity of 100%, a species differentiation rate of 81%, and a cross reactivity of 6%.

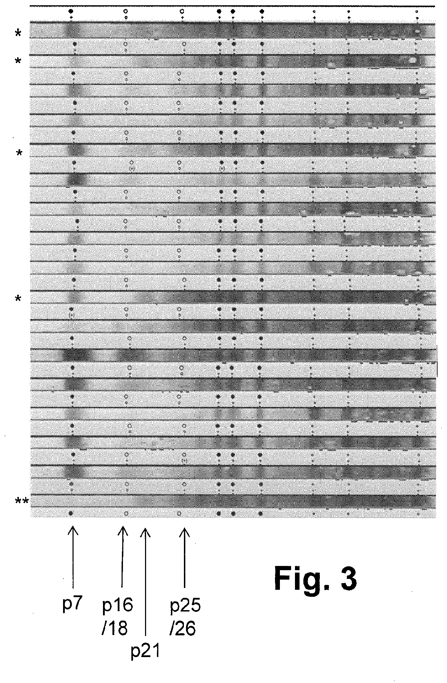

In several cases, exemplified by the sample analyzed in the bottom channel depicted in **Fig. 3****,** an *Echinococcus* infection could be detected on the basis of antibodies to p21, whilst not antibodies to p7 or p16/18 could be detected.

## Claims

1. A diagnostically useful carrier comprising a means for specifically capturing an antibody to p21 from *Echinococcus multilocularis* in a sample from a subject, wherein the carrier further comprises
(a) means for specifically capturing antibodies to one or more polypeptides of p7 and p16/18 from *Echinococcus multilocularis,* and
(b) means for specifically capturing antibodies to one or more polypeptides of AgB8/1a set forth in SEQ ID NO:1, AgB8/1b set forth in SEQ ID NO:2, AgB8/2 set forth in SEQ ID NO:3, AgB8/3 set forth in SEQ ID NO:4, AgB8/4a set forth in SEQ ID NO:5 and AgB8/4b set forth in SEQ ID NO:6 from *Echinococcus granulosus,* and Em18 set forth in SEQ ID NO:7 and Em95 set forth in SEQ ID NO:8 from *Echinococcus multilocularis,*
wherein p21 is a polypeptide having an apparent molecular weight of 21 kDa as judged by SDS PAGE under reducing conditions.

2. The diagnostically useful carrier according to claim 1, wherein the carrier further comprises a means for specifically capturing an antibody to one or more polypeptides of p25/26 from *Echinococcus multilocularis.*

3. The diagnostically useful carrier according to any of claims 1 to 2, wherein the means for specifically capturing an antibody to a polypeptide from the group comprising p21, p7, p16/18 and p25/26 from *Echinococcus multilocularis* is the respective polypeptide from the group comprising p21, p7, p16/18 and p25/26 from *Echinococcus multilocularis* or a variant thereof, preferably in a native form of the polypeptide, wherein a variant is a polypeptide comprising amino acid sequences that are at least 90 % identical to the reference amino acid sequence referred to.

4. The diagnostically useful carrier according to any of claims 1 to 3, wherein the means for specifically capturing an antibody to a polypeptide from the group comprising p21, p7, p16/18 and p25/26 from *Echinococcus multilocularis* is provided in the form of a polypeptide extract from *Echinococcus,* preferably *Echinococcus multilocularis* separated according to molecular weight, wherein the polypeptide extract comprises p16/18 and p21 which are separated sufficiently to allow for distinction between an antibody to p16/18 and another antibody to p21.

5. The diagnostically useful carrier according to claim 1, wherein the means for specifically capturing an antibody to one or more polypeptides selected from the group comprising AgB8/1a, AgB8/1b, AgB8/2, AgB8/3, AgB8/4a and AgB8/4b from *Echinococcus granulosus,* Em18 and Em95 from *Echinococcus multilocularis* is the respective polypeptide from the group comprising AgB8/1a, AgB8/1b, AgB8/2, AgB8/3, AgB8/4a and AgB8/4b from *Echinococcus granulosus,* Em18 and Em95 from *Echinococcus multilocularis* or a variant thereof, preferably provided in the form of one or more recombinant and/or purified polypeptides, wherein a variant is a polypeptide comprising amino acid sequences that are at least 90 % identical to the reference amino acid sequence referred to.

6. A kit comprising the diagnostically useful carrier according to any of claims 1 to 5, preferably further comprising a means for detecting any captured antibody.

7. A method comprising the step detecting an antibody to p21 from *Echinococcus multilocularis* in a sample from a subject and further comprising detecting
(a) antibodies to one or more polypeptides of p7 and p16/18; and
(b) antibodies to one or more polypeptides of AgB8/1a set forth in SEQ ID NO:1, AgB8/1b set forth in SEQ ID NO:2, AgB8/2 set forth in SEQ ID NO:3, AgB8/3 set forth in SEQ ID NO:4, AgB8/4a set forth in SEQ ID NO:5 and AgB8/4b set forth in SEQ ID NO:6 from *Echinococcus granulosus,* and Em18 set forth in SEQ ID NO:7 and Em95 set forth in SEQ ID NO:8 from *Echinococcus multilocularis,*
wherein p21 is a polypeptide having an apparent molecular weight of 21 kDa as judged by SDS PAGE under reducing conditions.

8. The method according to claim 7, further comprising detecting an antibody to one or more polypeptides of p25/26.

9. The method according to claim 7 or 8, wherein antibodies to more than one of the polypeptides from said group are detected simultaneously.

10. The method according to any of claims 7 to 9, wherein the antibodies to more than one of the polypeptides are detected in spatially separate binding reactions or in a one-pot reaction, preferably in a one-pot reaction.

11. The method according to any of claims 7 to 10, wherein an antibody to p21 is detected separately and can be distinguished from an antibody binding to p16/18.

## Patentansprüche

1. Ein diagnostisch verwendbarer Träger umfassend ein Mittel zum spezifischen Einfangen eines Antikörpers gegen p21 aus *Echinococcus multilocularis* in einer Probe von einem Probanden, wobei der Träger ferner
(a) ein Mittel zum spezifischen Einfangen von Antikörpern gegen ein oder mehrere Polypeptide von p7 und p16/18 aus *Echinococcus multilocularis* und
(b) ein Mittel zum spezifischen Einfangen von Antikörpern gegen ein oder mehrere Polypeptide von AgB8/1a, wie aufgeführt in SEQ ID NO:1, AgB8/1b, wie aufgeführt in SEQ ID NO:2, AgB8/2, wie aufgeführt in SEQ ID NO:3, AgB8/3, wie aufgeführt in SEQ ID NO:4, AgB8/4a, wie aufgeführt in SEQ ID NO:5, und AgB8/4b, wie aufgeführt in SEQ ID NO:6, von *Echinococcus granulosus* und Em18, wie aufgeführt in SEQ ID NO:7, und Em95, wie aufgeführt in SEQ ID NO:8, von *Echinococcus multilocularis*
umfasst,
wobei p21 ein Polypeptid ist, das ein offenbares Molekulargewicht von 21 kDa hat, wenn es durch eine SDS-PAGE unter reduzierenden Bedingungen eingeschätzt wird.

2. Der diagnostisch verwendbare Träger nach Anspruch 1, wobei der Träger ferner ein Mittel zum spezifischen Einfangen eines Antikörpers gegen ein oder mehrere Polypeptide von p25/26 aus *Echinococcus multilocularis* umfasst.

3. Der diagnostisch verwendbare Träger nach einem der Ansprüche 1 bis 2, wobei das Mittel zum spezifischen Einfangen eines Antikörpers gegen ein Polypeptid aus der Gruppe umfassend p21, p7, p16/18 und p25/26 aus *Echinococcus multilocularis* das jeweilige Polypeptid aus der Gruppe umfassend p21, p7, p16/18 und p25/26 aus *Echinococcus multilocularis* oder eine Variante davon ist, bevorzugt in einer nativen Form des Polypeptids, wobei eine Variante ein Polypeptid ist, das Aminosäuresequenzen umfasst, die zu mindestens 90% identisch zu der betreffenden Referenz-Aminosäuresequenz sind.

4. Der diagnostisch verwendbare Träger nach einem der Ansprüche 1 bis 3, wobei das Mittel zum spezifischen Einfangen eines Antikörpers gegen ein Polypeptid aus der Gruppe umfassend p21, p7, p16/18 und p25/26 aus *Echinococcus multilocularis* das in Form eines Polypeptid-Extraktes aus *Echinococcus,* bevorzugt aus *Echinococcus multilocularis,* bereitgestellt und nach Molekulargewicht aufgetrennt wird, wobei der Polypeptid-Extrakt p16/18 und p21 umfasst, die ausreichend aufgetrennt sind, um eine Unterscheidung zwischen einem Antikörper gegen p16/18 und einem anderen Antikörper gegen p21 zu ermöglichen.

5. Der diagnostisch verwendbare Träger nach Anspruch 1, wobei das Mittel zum spezifischen Einfangen eines Antikörpers gegen ein oder mehrere Polypeptide ausgewählt aus der Gruppe, die AgB8/1a, AgB8/1b, AgB8/2, AgB8/3, AgB8/4a und AgB8/4b aus *Echinococcus granulosus,* Em18 und Em95 aus *Echinococcus multilocularis* umfasst, das jeweilige Polypeptid aus der Gruppe, die AgB8/1a, AgB8/1b, AgB8/2, AgB8/3, AgB8/4a und AgB8/4b aus *Echinococcus granulosus,* Em18 und Em95 aus *Echinococcus multilocularis* oder eine Variante davon umfasst, ist, bevorzugt bereitgestellt in der Form eines oder mehrerer rekombinanter und/oder aufgereinigter Polypeptide, wobei eine Variante ein Polypeptid ist, das Aminosäuresequenzen umfasst, die zu mindestens 90% identisch zu der betreffenden Referenz-Aminosäuresequenz sind.

6. Ein Kit umfassend den diagnostisch verwendbaren Träger nach einem der Ansprüche 1 bis 5, wobei es bevorzugt ferner ein Mittel zum Nachweisen jedes eingefangenen Antikörpers umfasst.

7. Ein Verfahren umfassend den Schritt Nachweisen eines Antikörpers gegen p21 aus *Echinococcus multilocularis* in einer Probe von einem Probanden und ferner umfassend das Nachweisen von
(a) Antikörpern gegen ein oder mehrere Polypeptide von p7 und p16/18; und
(b) Antikörpern gegen ein oder mehrere Polypeptide von AgB8/1a, wie aufgeführt in SEQ ID NO:1, AgB8/1b, wie aufgeführt in SEQ ID NO:2, AgB8/2, wie aufgeführt in SEQ ID NO:3, AgB8/3, wie aufgeführt in SEQ ID NO:4, AgB8/4a, wie aufgeführt in SEQ ID NO:5, und AgB8/4b, wie aufgeführt in SEQ ID NO:6, von *Echinococcus granulosus* und Em18, wie aufgeführt in SEQ ID NO:7, und Em95, wie aufgeführt in SEQ ID NO:8, aus *Echinococcus multilocularis,*
wobei p21 ein Polypeptid ist, das ein offenbares Molekulargewicht von 21 kDa hat, wenn es durch eine SDS-PAGE unter reduzierenden Bedingungen eingeschätzt wird.

8. Das Verfahren nach Anspruch 7 ferner umfassend das Nachweisen eines Antikörpers gegen ein oder mehrere Polypeptide von p25/26.

9. Das Verfahren nach Anspruch 7 oder 8, wobei Antikörper gegen mehr als eines der Polypeptide aus der genannten Gruppe gleichzeitig nachgewiesen werden.

10. Das Verfahren nach einem der Ansprüche 7 bis 9, wobei die Antikörper gegen mehr als eines der Polypeptide in räumlich getrennten Bindungsreaktionen oder in einer Eintopfreaktion, bevorzugt in einer Eintopfreaktion, nachgewiesen werden.

11. Das Verfahren nach einem der Ansprüche 7 bis 10, wobei ein Antikörper gegen p21 getrennt nachgewiesen wird und von einer Antikörperbindung gegen p16/18 unterschieden werden kann.

## Revendications

1. Véhicule utile en diagnostic comprenant un moyen pour capturer spécifiquement un anticorps contre p21 d'*Echinococcus multilocularis* dans un échantillon d'un sujet, le véhicule comprenant en outre
(a) un moyen pour capturer spécifiquement des anticorps contre un ou plusieurs polypeptides de p7 et p16/18 d'*Echinococcus multilocularis,* et
(b) un moyen pour capturer spécifiquement des anticorps contre un ou plusieurs polypeptides d'AgB8/1a décrit dans SEQ ID NO: 1, AgB8/1b décrit dans SEQ ID NO: 2, AgB8/2 décrit dans SEQ ID NO: 3, AgB8/3 décrit dans SEQ ID NO: 4, AgB8/4a décrit dans SEQ ID NO: 5 et AgB8/4b décrit dans SEQ ID NO: 6 d'*Echinococcus granulosus,* et Em18 décrit dans SEQ ID NO: 7 et Em95 décrit dans SEQ ID NO: 8 d'*Echinococcus multilocularis,*
dans lequel p21 est un polypeptide ayant un poids moléculaire apparent de 21 kDa comme indiqué par SDS PAGE dans des conditions réductrices.

2. Véhicule utile en diagnostic selon la revendication 1, le véhicule comprenant en outre un moyen pour capturer spécifiquement un anticorps contre un ou plusieurs polypeptides de p25/26 d'*Echinococcus multilocularis.*

3. Véhicule utile en diagnostic selon l'une quelconque des revendications 1 à 2, dans lequel le moyen pour capturer spécifiquement un anticorps contre un polypeptide du groupe comprenant p21, p7, p16/18 et p25/26 d'*Echinococcus multilocularis* est le polypeptide respectif du groupe comprenant p21, p7, p16/18 et p25/26 d'*Echinococcus multilocularis* ou un variant de celui-ci, de préférence sous une forme native du polypeptide, un variant étant un polypeptide comprenant des séquences d'acides aminés qui sont au moins 90 % identiques à la séquence d'acides aminés de référence référencée.

4. Véhicule utile en diagnostic selon l'une quelconque des revendications 1 à 3, dans lequel le moyen pour capturer spécifiquement un anticorps contre un polypeptide du groupe comprenant p21, p7, p16/18 et p25/26 d'*Echinococcus multilocularis* est fourni sous la forme d'un extrait de polypeptides d'*Echinococcus,* de préférence *Echinococcus multilocularis,* séparés en fonction du poids moléculaire, dans lequel l'extrait de polypeptides comprend p16/18 et p21 qui sont suffisamment séparés pour permettre la distinction entre un anticorps contre p16/18 et un autre anticorps contre p21.

5. Véhicule utile en diagnostic selon la revendication 1, dans lequel le moyen pour capturer spécifiquement un anticorps contre un ou plusieurs polypeptides choisis dans le groupe comprenant AgB8/1a, AgB8/1b, AgB8/2, AgB8/3, AgB8/4a et AgB8/4b d'*Echinococcus granulosus,* Em18 et Em95 d'*Echinococcus multilocularis* est le polypeptide respectif du groupe comprenant AgB8/1a, AgB8/1b, AgB8/2, AgB8/3, AgB8/4a et AgB8/4b d'*Echinococcus granulosus,* Em18 et Em95 d'*Echinococcus multilocularis* ou un variant de celui-ci, de préférence fourni sous la forme d'un ou plusieurs polypeptides recombinants et/ou purifiés, dans lequel un variant est un polypeptide comprenant des séquences d'acides aminés qui sont au moins 90 % identiques à la séquence d'acides aminés de référence référencée.

6. Trousse comprenant le véhicule utile en diagnostic selon l'une quelconque des revendications 1 à 5, de préférence comprenant en outre un moyen pour détecter un anticorps capturé quelconque.

7. Procédé comprenant l'étape de détection d'un anticorps contre p21 d'*Echinococcus multilocularis* dans un échantillon d'un sujet et comprenant en outre la détection de
(a) anticorps contre un ou plusieurs polypeptides de p7 et p16/18 ; et
(b) anticorps contre un ou plusieurs polypeptides d'AgB8/1a décrit dans SEQ ID NO: 1, AgB8/1b décrit dans SEQ ID NO: 2, AgB8/2 décrit dans SEQ ID NO: 3, AgB8/3 décrit dans SEQ ID NO: 4, AgB8/4a décrit dans SEQ ID NO: 5 et AgB8/4b décrit dans SEQ ID NO: 6 d'*Echinococcus granulosus,* et Em18 décrit dans SEQ ID NO: 7 et Em95 décrit dans SEQ ID NO: 8 d'*Echinococcus multilocularis,*
dans lequel p21 est un polypeptide ayant un poids moléculaire apparent de 21 kDa comme indiqué par SDS PAGE dans des conditions réductrices.

8. Procédé selon la revendication 7, comprenant en outre la détection d'un anticorps contre un ou plusieurs polypeptides de p25/26.

9. Procédé selon la revendication 7 ou 8, dans lequel des anticorps contre plusieurs polypeptides dudit groupe sont détectés simultanément.

10. Procédé selon l'une quelconque des revendications 7 à 9, dans lequel les anticorps contre plusieurs polypeptides sont détectés dans des réactions de liaison spatialement séparées ou dans une réaction monopote, de préférence dans une réaction monopote.

11. Procédé selon l'une quelconque des revendications 7 à 10, dans lequel un anticorps contre p21 est détecté séparément et peut être distingué d'un anticorps se liant à p16/18.
